# EUROPEAN PATENT SPECIFICATION

(11) **EP 1 596 851 B1**
(45) Date of publication and mention of the grant of the patent: **03.01.2007**
(21) Application number: 04706141.1
(22) Date of filing: 29.01.2004
(51) Int. Cl.: A61K 31/185, A61P 11/00, A61P 31/12, A61P 31/16

(54) **THE USE OF SODIUM 2-MERCAPTOETHANESULFONATE AS ANTIVIRAL AGENT**
VERWENDUNG VON NATRIUM-2-MERCAPTOETHANSULPHONAT ALS ANTIVIRALES MITTEL
UTILISATION DE 2-MERCAPTO-ETHANE SULFONATE DE SODIUM EN TANT QU'AGENT ANTIVIRAL

(30) Priority: 04.02.2003 IT MI20030175
(43) Date of publication of application: 23.11.2005
(73) Proprietor: Piazza, Fabio, 43100 Parma (IT); Conti, Giorgio, 43019 Soragna (IT); Portincasa, Pietro, 43035 S. Ilario Baganza (IT); Zini, Carlo, 43100 Parma (IT)
(72) Inventor: Piazza, Fabio, 43100 Parma (IT); Conti, Giorgio, 43019 Soragna (IT); Portincasa, Pietro, 43035 S. Ilario Baganza (IT); Zini, Carlo, 43100 Parma (IT)
(74) Representative: Minoja, Fabrizio
(86) International application number: PCT/EP2004/000779
(87) International publication number: WO 2004/069235

(56) References cited:
- EP-B- 0 930 878
- WO-A-01/42780
- DE-A- 3 728 917
- US-A- 3 567 835
- DATABASE BIOSIS [Online] BIOSCIENCES INFORMATION SERVICE, PHILADELPHIA, PA, US; 1980 SEMCZUK B ET AL: "ASSESSMENT OF CLINICAL USEFULNESS OF MESNA MUCO FLUID MISTABRON IN TREATMENT OF CERTAIN LARYNGOLOGICAL DISEASES" Database accession no. PREV198172069103 XP002280281 & OTOLARYNGOLOGIA POLSKA, vol. 34, no. 6, 1980, pages 553-558, ISSN: 0030-6657
- DATABASE EMBASE [Online] ELSEVIER SCIENCE PUBLISHERS, AMSTERDAM, NL; 1997 DAMBORENEA TEJADA J ET AL: "Outcome of the treatment of chronical unespecific faringitis with mesna and aerosoltherapy" Database accession no. EMB-1997232341 XP002280282 & O.R.L.-DIPS 1997 SPAIN, vol. 24, no. 3, 1997, pages 102-106, ISSN: 0210-7309
- DATABASE EMBASE [Online] ELSEVIER SCIENCE PUBLISHERS, AMSTERDAM, NL; 1995 MOTTA G ET AL: "Clinical evaluation of mesna in hypersecreting rhinitis" Database accession no. EMB-1995161436 XP002280283 & RIVISTA ITALIANA DI OTORINOLARINGOLOGIA AUDIOLOGIA E FONIATRIA 1995 ITALY, vol. 15, no. 1, 1995, pages 61-65, ISSN: 0392-1360
- CORDERO L ET AL: "Protocols for minor ailments of the TESEMED project: Cold and flu" PHARMACEUTICAL CARE ESPANA 2001 SPAIN, vol. 3, no. 1, 2001, pages 5-21, XP008029738 ISSN: 1139-6202
- BONIVER R.: "Utilisation de l'UCB 3983 dans le traitement des otites séreuses" BRUXELLES MEDICAL, vol. 53, no. 6, 1973, pages 395-396, XP008029745
- BEERS M. AND BERKOW R.: "The Merck Manual of Diagnosis and Therapy - Seventeenth Edition" 1999 , MERCK RESEARCH LABORATORIES , WHITEHOUSE STATION, N.J. XP002280280 page 685, column 2, paragraph 2 page 687, column 2, paragraph 4 page 690, column 1, paragraph 3 page 693, column 2, paragraph 2
- BEERS M. AND BERKOW R.: "The Merck Manual of Diagnosis and Therapy - Seventeenth Edition" 1999 , MERCK RESEARCH LABORATORIES , WHITEHOUSE STATION, N.J. XP002280351 page 1277 -page 1293, column 2, paragraph 1

## Description

### FIELD OF THE INVENTION

The present invention relates to the use of sodium 2-mercaptoethanesulfonate as anti-influenza agent in human and/or veterinary medicine.

### PRIOR ART

Sodium 2-mercaptoethanesulfonate (hereinafter also referred to as MESNA) has been used for some time as mucolytic agent, thanks to its ability to cleave the disulfide bridges in the polypeptide chains of the mucus, for the treatment of pathological conditions of the respiratory system, such as chronic bronchitis, asthma, emphysema, cystic fibrosis and rhinitis (see e.g. US 3 567 835; Semczuk et al., Otolaryngologia Polska, 34(6), 1980, 553-558; Damborenea Tejada et al., O.R.L.-DIPS, 24(3), 1997, 102-106; Motta et al., Rivista Italiana di Otorinolaringologia Audiologia e Foniatria, 15(1), 1995, 61-65), and in post-surgery in combination with respiratory physiotherapy, to promote expectoration and to prevent pulmonary complications. Furthermore, MESNA is used in oncology for the prevention of lesions of the urinary tracts due to anticancer agents such as cyclophosphamide (see e.g. EP 591 710 and EP 334 083).

Finally, EP 930 878 discloses the use of MESNA in surgery, in the form of topical formulations, in particular sterile solutions, for the direct application during surgery of normal and/or pathologic tissues, in order to facilitate dissection.

In WO 01/42780, it has been demonstrated that MESNA is able to disassemble papillomavirus virus-like particles in vivo after prolonged exposure to high concentrations of MESNA.

It has now been found that MESNA exerts a remarkable action against the influenza viruses. Therefore the present invention relates to the use of MESNA in the treatment of influenza virus infections.

### PHARMACOLOGICAL EXPERIMENTATION

The "in vitro" effect of MESNA on the multiplicative cycle of two negative-strand RNA viruses: human influenza A virus NWS/33, H1N1, and avian influenza A virus Ulster/73, H7N1, belonging to the Orthomyxoviridae family, has been studied.

### Materials and methods

### Cell cultures and virus strains

Monolayer cultures of LLC monkey kidney epithelial cells (LLC-MK2) and Madin Darby canine kidney (NOCK) cells were grown in Minimum Essential Medium with Earle's salts (M.E.M. Earle's) supplemented with foetal calf serum to 10% final concentration. The human neurotropic variant, NWS strain (H1N1), of type A influenza virus was grown in the allantoic cavity of fertile hen eggs. After 48 hours growth the allantoic fluid was harvested, centrifuged at 5.000 rpm for 30 minutes to remove cellular debris, titered by haemagglutination with human group O Rh+ red blood cells, plaque assayed and used as inoculum for all the experiments.

The results obtained with human influenza strain were further confirmed and supported by using also type A influenza virus, Ulster/73, H7N1, which is a model of avian influenza virus.

### Effect of MESNA-treatment on viral growth

Confluent monolayers of LLC-MK2 cells were infected with different dilutions of A/NWS/33. Virus in phosphate buffer (P.B.S.) pH 7.4. After 45 minute adsorption, LLC-MK2 cellular monolayers were treated with different amounts of MESNA to define the concentration of the compound completely inhibiting viral growth; the amounts of the compound used were 0.5, 1, 2, 5, 10, 15, 20 mg/ml, added to the maintenance medium from the beginning of infection and left during the whole experiment. After 12, 24, 36, 48, 60, 72 hours post infection (p.i.) the supernatant of infected and treated cell monolayers was collected, centrifuged at 2.500 rpm to remove cell debris and assayed by haemagglutination (presence/absence of mature viral particles).

MESNA proved able to suppress virus production in LLC-NM cells at all the tested concentrations.

In a different set of experiments, MESNA did not interfere with virus-specific receptors located on red blood cells used for the haemagglutination assay.

### Post-treatment experiments

Post-treatment experiments were performed to elucidate which stage of virus replication was mainly affected by MESNA. At different time-points after the beginning of infection, MESNA was added to the maintenance medium of infected-LLC-MK2 cell monolayers and left until 24, 48, 72 hours post infection. The production of viral particles was evaluated by haemagglutination titration.

Addition of MESNA proved effective until 6 hours post infection.

### Reversibility

Experiments of reversibility were also performed to elucidate the general characteristics of MESNA-target binding.

Cellular monolayers of LLC-MK2 cells were infected and treated with MESNA; then the maintenance medium containing MESNA was discarded at 1, 2, 4, 5, 6, 8, 10 hours p.i. and maintenance medium without MESNA was added and left until 24, 48, 72 hours post infection. HA titrations were carried out after 24, 48 and 72 hours of infection: MESNA exhibited an irreversible inhibition on the production of viral particles at all the tested times.

### Haemadsorption assay on MESNA-treated infected LLC-MK2 cells

This assay was carried out to verify the proper biological role of the haemagglutinin viral polypeptide (HA), because a correct haemadsorption of red blood cells correlates not only with the integrity of the glycosylated haemagglutinin molecule but also with its correct insertion into the cellular membranes of infected cells.

The presence of viral haemagglutinin HA antigen on the cellular surface of infected monolayers of LLC-MK2 cells treated or untreated with MESNA was monitored at 24 and 48 hours post infection by haemadsorption assay performed according to the Finter technique. As a result, significant differences between untreated versus treated infected cells were observed. The obtained haemadsorption values indicate a great reduction, if not the absence, of haemagglutinin molecules on the surface of infected and MESNA-treated cells.

### Effect of MESNA-treatment on virus protein synthesis

Confluent monolayers of LLC-MK2 cells were infected by A/NWS/33; after virus inoculum was discarded, infected cells were treated with MESNA at 20 mg/ml. At different times after the infection, confluent cellular monolayers were labeled with [³⁵S]-methionine (20 µCi/ml) in methionine-free medium containing 2% dialysed foetal calf serum. Cells were usually preincubated for 40 minutes starvation period in methionine-free medium.

After 30 minutes of labelling, cells were washed in 0.9% sterile NaCl solution (Puck's saline), scraped off with a rubber Teflon policeman, pelleted by centrifugation at 2.500 xg for 5 minutes and then lysed with Laemmli's lysis buffer before electrophoresis onto 15% S.D.S-polyacrylamide slab gel at 50 Volts. Virus-induced polypeptides were visualized by autoradiography on Kodak X-ray films. MESNA-treatment determined a marked reduction of the normal synthesis of virus-induced polypeptides: both the synthesis of early as well as late viral proteins were greatly inhibited. The inhibiting effect was particularly evident when using amounts of MESNA able to block virus production, namely 1, 2, 5, 1.0, 15 mg/ml.

### Comparison studies

A separate set of experiments was also performed, using the commercial compound MUCOLENE, which consists of MESNA plus sodium edetate. The results were comparable to those obtained with MESNA alone. To evidence any possible, even synergistic, action performed by sodium edetate, all the experiments were repeated with sodium edetate alone: the obtained results demonstrated that sodium edetate was not able to exert an antiviral action at any concentration tested.

### Conclusions

Results obtained with avian influenza virus A/Ulster/73, H7N1 strain were completely reproducible and absolutely comparable with those obtained with human influenza virus, A/NWS/33, H1N1 strain, as described above.

It should also be stressed that the antiviral action is exerted through inhibition not only of the production of mature viral particles, but also of the synthesis of any virus-specific proteins.

The results of the pharmacological experimentation were confirmed by clinical tests on volunteers.

According to the present invention, MESNA will be administered in an effective amount, i.e. an amount sufficient to attain the antiviral prophylactic or therapeutical effect desired.

The amount of MESNA necessary to obtain the desired antiviral effect will depend on a number of factors, such as type of disease, route of administration and conditions of the patient. As a rule, a typical daily dosage will range from 5 mg/kg to 60 mg/kg, administered as a single unitary dose or as separated repeated doses.

MESNA will be formulated in combination with pharmaceutically acceptable carriers and excipients. According to the present invention, the pharmaceutical or veterinary compositions containing MESNA will be presented in forms suitable for the oral, sublingual, buccal, parenteral, rectal, topical, intranasal, inhalatory and transdermal administrations.

Examples of formulations for the oral, sublingual and buccal administration comprise tablets, capsules, sugar-coated pills, lozenges, powders, granules, solutions, suspensions and emulsions. Examples of formulations for the parenteral administration comprise sterile solutions and emulsions for injection (for example subcutaneous, intramuscular, or intravenous). Examples of formulations for the rectal administration are suppositories. Examples of formulations for the intranasal and inhalatory administration comprise spray, aerosol, nebulizers and pressurized metered inhalers.

Particularly preferred are topical formulations for the first aereodigestive tract, for example topical nasal, pharyngeal, rhino-pharyngeal, oro-pharyngeal forms, such as collutories, gurgles and mouth-washes.

The pharmaceutical compositions containing MESNA may also contain other physiologically active ingredients.

The formulations will be prepared according to conventional procedures of pharmaceutical technique, using suitable excipients and excipients, such as diluents, preservatives, antioxidants, flavours, thickening agents, isotonicity agents, buffer agents, surfactants, flavours and the like.

## Claims

1. The use of sodium 2-mercaptoethanesulfonate for the preparation of a pharmaceutical composition for the treatment and the prevention of influenza virus infections.

2. The use as claimed in claim 1, wherein the composition is to be administered by the topical nasal, pharyngeal, rhinopharyngeal, oro-pharyngeal routes.

## Patentansprüche

1. Verwendung von Natrium-2-mercaptoethansulfonat zur Herstellung einer pharmazeutischen Zusammensetzung zur Behandlung von und der Prävention gegen Influenzavirusinfektionen.

2. Verwendung nach Anspruch 1, wobei die Zusammensetzung zu verabreichen ist über den topisch nasalen, pharyngealen, rhinopharyngealen oder oro-pharyngealen Verabreichungsweg.

## Revendications

1. Utilisation de 2-mercaptoéthanesulfonate de sodium pour la préparation d'une composition pharmaceutique pour le traitement et la prévention d'infections par le virus influenza.

2. Utilisation selon la revendication 1, dans laquelle la composition est à administrer par les voies topiques nasale, pharyngienne, rhino-pharyngienne, oro-pharyngienne.
